(19) **Europäisches Patentamt / European Patent Office / Office européen des brevets**

(11) **EP 2 695 526 A1**

(12) **EUROPEAN PATENT APPLICATION**
published in accordance with Art. 153(4) EPC

(43) Date of publication:
**12.02.2014 Bulletin 2014/07**

(21) Application number: **12768006.4**

(22) Date of filing: **04.04.2012**

(51) Int Cl.:
*A23L 1/227* (2006.01)     *A23L 1/221* (2006.01)

(86) International application number:
**PCT/JP2012/059209**

(87) International publication number:
**WO 2012/137825 (11.10.2012 Gazette 2012/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.04.2011   JP 2011083155**

(71) Applicant: **Ajinomoto Co., Inc.
Tokyo, 104-8315 (JP)**

(72) Inventors:
• **KITAJIMA, Seiji
  Kawasaki-shi, Kanagawa 210-8681 (JP)**
• **ETO, Yuzuru
  Kawasaki-shi, Kanagawa 210-8681 (JP)**
• **TAJIMA, Takaho
  Kawasaki-shi, Kanagawa 210-8681 (JP)**

(74) Representative: **Strehl Schübel-Hopf & Partner
Maximilianstraße 54
D-80538 München (DE)**

(54) **MANUFACTURING METHOD FOR FOOD WITH ENHANCED TASTE AND METHOD FOR ENHANCING TASTE OF FOOD**

(57)    The present invention aims to provide a production method of a food, which can be used more generally and can effectively enhance a taste without conferring an unpreferable flavor, and a method for enhancing a taste of food. The present invention provides a method of producing a food with an enhanced taste, including a step of adding a peptide mixture obtained by hydrolyzing, with an enzyme, a composition containing β-conglycinin, and a method of enhancing the taste of food, including a step of adding the peptide mixture.

**EP 2 695 526 A1**

**Description**

Technical Field

The present invention relates to a method of producing a food with an enhanced taste, which comprises using a peptide mixture that can be obtained by hydrolyzing a composition containing β-conglycinin, and a method for enhancing the taste of food.

Background Art

Soybean contains many proteins with good quality, and has been widely utilized as a superior protein source. *Miso* and soysauce utilizing a hydrolysate of soybean protein can season food at a low cost, and have been widely used as basic seasonings capable of improving the taste of food. However, since a hydrolysate of soybean protein itself also has an unpreferable flavor, there is a problem of conferring an undesired taste in seasoning a food.

As a method for enhancing the basic taste without losing the balance of the taste of food, improvement of taste with *kokumi* is known. *Kokumi* is a taste that cannot be expressed by the five basic tastes (sweet taste, salty taste, sour taste, bitter taste and *umami),* and has effects of not only enhancing the basic tastes but also enhancing marginal tastes of the basic tastes such as thickness, growth (mouthfulness), continuity, harmony and the like of the taste. Conventionally, some methods for conferring *kokumi* have been reported, and methods of adding glutathione (patent document 1), glycopeptide (patent document 2), dipeptide and tripeptide (non-patent document 1, non-patent document 2) and the like are known. However, these methods for conferring *kokumi* have a problem in that it cannot be provided at a low cost, and the like.

Soybean protein is made of various proteins having macromolecular complex structures, and can be divided, for example, into proteins such as 2S, 7S, 11S, 15S and the like by a fractionation method based on the difference in the sedimentation coefficient by ultracentrifugation analysis. These proteins have various, different characteristics not only in physical properties but also function. For example, β-conglycinin, which is the main component of 7S, has been reported to have a blood triglyceride improving effect (non-patent document 3), and a soybean protein mixture wherein β-conglycinin contained in soybean protein has been selectively decomposed has been reported to improve physical properties such as improved emulsifiability and the like (patent document 3). In addition, a peptide mixture obtained by hydrolyzing β-conglycinin was examined for aggregation property (non-patent document 4), and further, physiological functions such as feeding suppressive action and the like have been reported (patent document 4, non-patent document 5). However, an examination case relating to a taste enhancing effect using a hydrolysate of β-conglycinin does not exist, and also, an examination case relating to a *kokumi* conferring effect thereof does not exist.

Document List

patent documents

patent document 1: Japan JP-B-1464928
patent document 2: WO2004/096836
patent document 3: Japan JP-B-3417350
patent document 4: WO2006/132273

non-patent documents

non-patent document 1: Journal of Agriculture and Food Chemistry, 2007, Vol.55, 6712-6719
non-patent document 2: Journal of Biological Chemistry, 2010, Vol.285, No.2, 1016-1022
non-patent document 3: Journal of Atherosclerosis and Thrombosis, 2006, Vol.13, No.5, 486-490
non-patent document 4: Journal of Agriculture and Food Chemistry, 1984, Vol. 32, 486-490
non-patent document 5: Biochemical and Molecular Action of Nutrients, 2003, Vol.133, 352-357

SUMMARY OF THE INVENTION

Problems to be Solved by the Invention

Under the background of the prior art described above, the present invention aims to provide a production method of a food, which can be used more generally and can effectively enhance a taste without conferring an unpreferable flavor, and a method for enhancing a taste of food.

Means of Solving the Problems .

The present inventors have conducted intensive studies in an attempt to solve the aforementioned problems and found that a peptide mixture obtained by enzymatic hydrolysis of β-conglycinin, which is a 7S component of soybean protein, which further contains a peptide having a protein molecular weight in a particular range as measured by the gel filtration method, and has a given trichloroacetic acid (0.22M) solubilization rate, provides an effect of enhancing the taste of food, particularly an effect of conferring kokumi to food, which resulted in the completion of the present invention.
Accordingly, the present invention contains the following.

[1] A method of producing a food with an enhanced taste, comprising a step of adding a peptide mixture obtained by hydrolyzing, with an enzyme, a composition comprising β-conglycinin as a main component, which mixture comprises a peptide having a protein molecular weight in a range of 2.6 - 9.4 kDa as measured by a gel filtration method, and shows a  trichloroacetic acid (0.22M) solubilization rate of 12.1 - 25.4%.
[2] The production method of [1], wherein the peak area of the peptide having a protein molecular weight in the range of 2.6 - 9.4 kDa as measured by a gel filtration method is 4.5 - 42.0% of the total peak area.
[3] The production method of [1] or [2], wherein the hydrolysis is performed under the conditions of pH 2 - 11 and/or 4 - 70°C.
[4] The production method of any one of [1] - [3], wherein the composition comprises not less than 80 wt% of β-conglycinin.
[5] The production method of any one of [1] - [4], wherein the composition is produced from a soybean with a high content of β-conglycinin as a starting material.
[6] The production method of [5], wherein the soybean with a high content of β-conglycinin is a soybean species containing 65 mg/g - 400 mg/g of β-conglycinin in a dry weight.
[7] A method of producing a food with an enhanced taste, comprising a step of adding a peptide mixture obtained by grinding and deoiling a soybean with a high content of β-conglycinin to give an extract, and hydrolyzing the extract with an enzyme under the conditions of pH 2 - 11 and/or 4-70°C.
[8] The production method of any one of [1] - [7], wherein the enzyme is one or more kinds selected from the group consisting of bromelain, papain, protease M, trypsin and aroase.
[9] The production method of any one of [1] - [7], wherein the enzyme is one or more kinds selected from the group consisting of bromelain, papain and protease M.
[10] The production method of any one of [1] - [9], wherein the peptide mixture is added at 0.001 - 99 wt%.
[11] A food composition with an enhanced taste, which is obtained by the production method of any one of [1] [10].
[12] The food composition of [11], which is a seasoning.
[13] A method for enhancing a taste of food, comprising a step of adding a peptide mixture obtained by hydrolyzing a  composition comprising β-conglycinin as a main component with an enzyme.
[14] The method of [13], wherein the composition comprises not less than 80 wt% of β-conglycinin.
[15] The method of [13] or [14], wherein the peptide mixture comprises a peptide having a protein molecular weight in a range of 2.6 - 9.4 kDa as measured by a gel filtration method.
[16] The method of any one of [13] - [15], wherein the peptide mixture has a trichloroacetic acid (0.22M) solubilization rate of 12.1 - 25.4%.
[17] The method of any one of [13] - [16], wherein the enzyme is one or more kinds selected from the group consisting of bromelain, papain, protease M, trypsin and aroase.
[18] The method of any one of [13] - [17], wherein the composition is prepared from a soybean with a high content of β-conglycinin as a starting material.
[19] The method of any one of [13] - [18], wherein the method for enhancing a taste is a method for conferring kokumi.
[20] A peptide mixture obtained by grinding and deoiling a soybean with a high content of β-conglycinin to give an extract, and hydrolyzing the extract with an enzyme under the conditions of pH 2 - 11 and/or 4 - 70°C.
[21] A taste enhancing agent comprising a peptide mixture obtained by hydrolyzing, with an enzyme, a composition comprising not less than 80 wt% of β-conglycinin, which mixture comprises a peptide having a protein molecular weight in a range of 2.6 - 9.4 kDa as measured by a gel filtration method, and shows a trichloroacetic acid (0.22M) solubilization rate of 12.1 - 25.4%, or the peptide mixture of [20].

[22] A taste enhancing agent comprising a peptide mixture obtained by hydrolyzing, with an enzyme, a composition comprising not less than 80 wt% of β-conglycinin, which mixture comprises a peptide having a protein molecular weight in a range of 2.6 - 9.4 kDa as measured by a gel filtration method, and shows a trichloroacetic acid (0.22M) solubilization rate of 12.1 - 25.4%.

[23] A food composition comprising a peptide mixture obtained by hydrolyzing, with an enzyme, a composition comprising not less than 80 wt% of β-conglycinin under the conditions of pH 2 - 11 and/or 4 - 70°C, which mixture

(1) comprises a peptide having a protein molecular weight in a range of 2.6 - 9.4 kDa as measured by a gel filtration method,
(2) shows a peak area of the peptide, which has a protein molecular weight in the range of 2.6 - 9.4 kDa as measured by a gel filtration method, of 4.5 - 42.0% of the total peak area, and
(3) shows a trichloroacetic acid (0.22M) solubilization rate of 12.1 - 25.4%.

Effect of the Invention

Using the peptide mixture derived from a β-conglycinin-containing composition, which is obtained by the present invention, the taste can be effectively enhanced without conferring an unpreferable flavor to a food, and *kokumi* can be conferred particularly effectively to food. In addition, since the peptide mixture can be prepared using general materials such as soybean and the like, it can be obtained at a low cost.

Brief Description of the Drawings

Fig. 1 shows the results of SDS electrophoresis of a hydrolysate of a β-conglycinin-containing composition, wherein the upper part of the stained gel shows the kind and concentration (μg/mL) of the enzyme used and the enzyme reaction time (min), and the left end shows the molecular weight (kDa) of the protein.
Fig. 2 shows the results of SDS electrophoresis of a soybean protein hydrolysate, wherein the upper part of the stained gel shows the concentration (μg/mL) of bromelain and the enzyme reaction time (min), and the left end shows the molecular weight (kDa) of the protein.
Fig. 3 shows the results of gel filtration chromatography analysis of hydrolysate C2 of a β-conglycinin-containing composition.
Fig. 4 shows the selection of fractions I - V based on the chart shown in Fig. 3, wherein the arrows show the fractionation starting points of fractions I-V.
Fig. 5 shows the results of SDS electrophoresis of fractions I - V, wherein the numeric values on the left end of the stained gel show the molecular weight (kDa) of the protein.
Fig. 6 shows a calibration curve obtained from the molecular weight of various protein markers and the elution time (retention time) measured by gel filtration chromatography, wherein the vertical axis of the graph shows the retention time (min) and the horizontal axis shows the molecular weight (kDa) of the protein.
Fig. 7 shows the relationship between the peak area rate in a protein molecular weight range of 2.6 - 9.4 kDa of hydrolysates C1-C6 of the β-conglycinin-containing composition, and a taste enhancing effect.
Fig. 8 shows the relationship between the trichloroacetic acid (TCA) solubilization rate of hydrolysates C1-C6 of the β-conglycinin-containing composition and a taste enhancing effect.

Description of Embodiments

β-conglycinin is a protein mainly contained in 7S globulin, which has a sedimentation coefficient of 7S by ultracentrifugation analysis, in globulin which is a generic name of soybean soluble globular proteins. Therefore, β-conglycinin is also referred to as 7S component. In the present invention, the content of β-conglycinin in the composition containing β-conglycinin as a main component is generally not less than 20 wt%, preferably not less than 40 wt%, more preferably not less than 50 wt%, further preferably not less than 80 wt%, and generally not more than 100 wt%, as a protein content. The composition may also contain not more than 95 wt% or not more than 90 wt% of β-conglycinin as a protein content. While the components other than β-conglycinin, in the composition are not particularly limited, examples thereof include proteins such as glycinin, lipophilic protein, hemagglutinin, trypsin inhibitor, lipoxynase and the like, phytic acid, saponin, isoflavone, linoleic acid, lecithin and the like, and they can be contained in the composition at a concentration set as appropriate. In addition to the above-mentioned components, the composition can contain, for example, water, organic solvents (alcohol such as ethanol, methanol etc., and the like), flavor, sugar, sweetener, fiber, vitamins, amino acids such as sodium glutamate and the like, nucleic acids such as inosine monophosphate and the like, inorganic salts such as sodium chloride etc., and the like.

A composition containing β-conglycinin as a main component (also to be referred to as "β-conglycinin-containing composition" in the present specification) can be produced by, for example, fractionating a soybean component by using soybean as a starting material. Examples of the production method thereof include the method of Thanh et al. (Journal of Agriculture and Food Chemistry, 1976, Vol. 24, 1117-1121), the method of Vagano et al. which is an improved method thereof (Journal of Agriculture and Food Chemistry, 1992, Vol. 40, 941-944), the method of Saito et al. (Bioscience, Biotechnology and Biochemistry, 2001, Vol. 65, 884-887) and the like.

As the starting material of the β-conglycinin-containing composition besides the above-mentioned soybean generally used in the food field, a soybean with a high content of β-conglycinin or an extract thereof is also preferably used. Specifically, the soybean with a high content of β-conglycinin, is preferably a soybean species containing about twice the amount of β-conglycinin as compared to ordinary soybean, for example, 65 mg/g - 400 mg/g, preferably 100 mg/g - 400 mg/g, in a dry weight. Examples of the soybean with a high content of β-conglycinin include, but are not limited to, "Nanahomare" (Nagano Vegetable and Ornamental Crops Experiment Station, The Hokuriku Crop Science, 2010, vol.45, 61-64) and the like.

When a soybean with a high content of β-conglycinin is used as a starting material, the β-conglycinin-containing composition is not particularly limited; however, it can be obtained as, for example, an extract of a ground soybean with a high content of β-conglycinin by a deoiling treatment thereof according to the method described in the below-mentioned Examples. Since this method unnecessitates a fractionation treatment of soybean protein and a purification treatment of β-conglycinin, it is preferable in the present invention.

The β-conglycinin-containing composition can be produced as mentioned above or a commercially available product can also be used preferably. Examples of the commercially available product of the β-conglycinin-containing composition include Lipoff-700 (manufactured by FUJI OIL CO., LTD.) and the like. In the present invention, moreover, β-conglycinin alone can also be used as the β-conglycinin-containing composition. As β-conglycinin, a commercially available product can be preferably used, and specific examples thereof include β-conglycinin (manufactured by Sigma Aldrich Ltd.) and the like.

In the present invention, the β-conglycinin-containing composition is directly, or after dissolution in a suitable solvent, subjected to hydrolysis with protease to give a peptide mixture. The thus-obtained peptide mixture is hereinafter sometimes to be referred to as the "peptide mixture of the present invention".

While the solvent used to dissolve the β-conglycinin-containing composition is not particularly limited as long as it is an aqueous solvent, for example, water, saline, sugar water, citrate buffer, acetic acid aqueous solution, alcohol solution and the like can be mentioned. When water is used, warm water can also be used, and the temperature at that time can be 4 - 100°C. While the concentration of β-conglycinin is not particularly limited, it is generally 0.01 - 20 wt%, preferably 0.05 - 10 wt%, more preferably 0.1 - 5 wt%, further preferably 0.5 - 2 wt%. The β-conglycinin-containing composition does not need to be completely dissolved in a solvent, and may be suspended partially or entirely.

The protease that hydrolyzes the β-conglycinin-containing composition is not particularly limited as long as it has the property of decomposing β-conglycinin to produce a peptide fragment. Examples of the protease include bromelain; papain; trypsin; proteases derived from the genus Aspergillus such as pepsin, pancreatin, protease P (manufactured by Amano Enzyme Inc.), protease M (manufactured by Amano Enzyme Inc.), Pantidase P (manufactured by YAKULT PHARMACEUTICAL INDUSTRY CO., LTD.) and the like; proteases derived from the genus Bacillus such as Thermoase, Neutrase (manufactured by Novozymes), aroase (manufactured by YAKULT PHARMACEUTICAL INDUSTRY CO., LTD.) and the like, and the like. A commercially available product selected appropriately can be used. In addition, two or more kinds of the above-mentioned proteases may be used in combination. Of these, bromelain, papain, protease M, trypsin and aroase are preferable, bromelain, papain, protease M and aroase are more preferable, and bromelain is particularly preferable, in the present invention, from the aspects of reactivity with β-conglycinin, a taste enhancing effect of the obtained peptide mixture and the like.

As the conditions for reacting a protease with the β-conglycinin-containing composition, conditions suitable for the protease to be used can be appropriately selected and set, since the optimal concentration, optimal temperature, optimal pH, reaction time and the like vary depending on the enzyme to be used. While these conditions are not particularly limited, the enzyme concentration is generally 0.0001 - 100 mg/mL, preferably 0.001 - 10 mg/mL, more preferably 0.01 - 1 mg/mL, the reaction temperature is generally 4 - 70°C, preferably 10 - 70°C, more preferably 15 - 70°C, the reaction pH is generally pH 2 - 11, preferably pH 2.5 - 10.5, more preferably pH 3 - 10, and the reaction time is generally 30 sec - 100 hr, preferably 1 min - 20 hr, more preferably 15 min - 360 min. In addition, in the case of using bromelain as a protease, the enzyme concentration is generally 0.0001 - 100 mg/mL, preferably 0.001 - 10 mg/mL, more preferably 0.01 - 1 mg/mL, the reaction temperature is generally 4 - 70°C, preferably 10 - 70°C, more preferably 15 - 70°C, the reaction pH is generally pH 2 - 11, preferably pH 2.5 - 10.5, more preferably pH 3 - 10, and the reaction time is generally 30 sec - 100 hr, preferably 1 min - 20 hr, more preferably 15 min - 360 min.

In the present invention, an inactivation treatment of protease is preferably performed after passage of a given reaction time. This prevents excessive hydrolysis of the β-conglycinin-containing composition, and can provide a peptide mixture of peptide fragments digested to have appropriate molecular weights. The method for the inactivation treatment is not particularly limited and a method known per se can be used. Examples thereof include a method of inactivation by heat denaturation such as boiling and the like, denaturation of enzyme by pH, a method by the addition of an enzyme reaction inhibitor, and the like.

In the present invention, a peptide mixture can be obtained by hydrolyzing the β-conglycinin-containing composition with an enzyme as mentioned above. While the hydrolysis is not particularly limited, it can be performed such that a trichloroacetic acid (0.22M) solubilization rate of the peptide mixture obtained using the β-conglycinin-containing composition is 12.1 - 25.4%, preferably 19.5 - 24.0%. Therefore, while the peptide mixture obtained by hydrolyzing the β-conglycinin-containing composition with an enzyme is not particularly limited, the trichloroacetic acid (0.22M) solubilization rate thereof is 12.1 - 25.4%, preferably 19.5 - 24.0%. The trichloroacetic acid solubilization rate is obtained by removing the precipitation of a high molecular weight protein component in a trichloroacetic acid solution and quantifying the low molecular weight protein component soluble in trichloroacetic acid, and can be used as an index showing the degree of protein hydrolysis (hydrolysis rate). While the measurement method of the trichloroacetic acid (0.22M) solubilization rate is not particularly limited, for example, the peptide mixture of the present invention is directly, or after dissolution or dispersion in a suitable solvent, added and mixed with a 0.44M trichloroacetic acid solution in an equal amount as the obtained solution, the mixture is incubated under the conditions of 37°C and 30 min, the precipitate is removed using a filter paper, the protein dissolved in the obtained filtrate is quantified, and the proportion relative to the total amount of the protein in the sample used is determined, whereby the rate can be measured. The solvent used for dissolving or dispersing the peptide mixture of the present invention is not particularly limited, and, for example, water, saline, sugar water, citrate buffer, aqueous acetic acid solution, alcohol solution and the like can be used. Also, the quantification of the protein is not particularly limited, and a method known per se, for example, Lowry method, Kjeldahl method and the like can be used.

When the peptide mixture of the present invention obtained above is preserved as a solution, it is preferably preserved at -20 - 4°C. In addition, the solution can also be freeze-dried and preserved in a powder state. When the peptide mixture is in a powder state, it is preferably preserved at -20 - 4°C, and preferably preserved together with a desiccant such as silica gel and the like. When the peptide mixture of the present invention is subjected to the below-mentioned gel filtration chromatography, the preserved peptide mixture of the present invention can be used directly or after dissolution in a suitable solvent. The solvent used for dissolving the peptide mixture of the present invention is not particularly limited and, for example, water, saline, sugar water, citrate buffer, aqueous acetic acid solution, alcohol solution and the like can be mentioned.

In the present invention, the peptide mixture of the present invention can contain a peptide having a protein molecular weight in a range of 2.6 - 9.4 kDa (in the present specification, said peptide means a mixture of peptides having a protein molecular weight in a range of 2.6 - 9.4 kDa). In the present invention, the range of the protein molecular weight can be measured by, for example, a gel filtration method, i.e., by subjecting the peptide mixture of the present invention to gel filtration chromatography. The gel filtration chromatography can be performed using a method known to those of ordinary skill in the art under, for example, the following conditions. Column: Superdex 75 10C/300 GL (manufactured by GE Healthcare), eluate: 20 mM phosphate buffer (pH 7.0), flow rate: 0.5 mL/min, column temperature: 25°C, detection: measurement of absorbance at wavelength 280 nm. As the analysis apparatus, high performance liquid chromatography apparatus, for example, high-performance liquid chromatography system LaChrom (manufactured by Hitachi, Ltd.) can be used.

The peptide within the above-mentioned range of the protein molecular weight in the peptide mixture of the present invention can be confirmed using a protein marker having a known molecular weight. For example, plural protein markers are analyzed under the same conditions as those for the gel filtration chromatography analysis of the peptide mixture of the present invention, a calibration curve is drawn from the obtained elution time and the molecular weights of various protein markers, and the range of elution time corresponding to the above-mentioned range of the protein molecular weight can be determined from the calibration curve. Using the obtained range of the elution time as the standard, and from the chart obtained by gel filtration chromatography analysis of the peptide mixture of the present invention, the peak of such peptide can be identified. The protein marker to be used is not particularly limited as long as it can prepare a calibration curve including the above-mentioned range of the protein molecular weight. For example, Conalbumin (75 kDa), bovine serum albumin (66 kDa), carbonic anhydrase (29 kDa), ovalbumin (43 kDa), ribonuclease A (13.7 kDa), Aprotinin (6.5 kDa), cyanocobalamin (1.36 kDa) and the like can be used in an appropriate combination. While these protein markers are not particularly limited, commercially available products can be preferably used, and a kit containing plural protein markers and the like can also be used. The range of the protein molecular weight can also be examined using other approach.

In addition, the peptide within the above-mentioned range of the protein molecular weight in the peptide mixture of the present invention may have, for example, a peak area obtained by gel filtration chromatography of 4.5 - 42.0%, more preferably 33 - 36%, of the total peak area. Here, the gel filtration chromatography is performed under the following conditions. Column: Superdex 75 100/300 GL (manufactured by GE Healthcare), eluate: 20 mM phosphate buffer (pH 7.0), flow rate: 0.5 mL/min, column temperature: 25°C, detection: measurement of absorbance at wavelength 280 nm. As the analysis apparatus, high performance liquid chromatography apparatus, for example, high-performance liquid chromatography system LaChrom (manufactured by Hitachi, Ltd.) can be used. The peak area can be determined from the chart obtained by gel filtration chromatography under the above conditions, and the peak area is set such that the range of the protein molecular weight is 2.6 - 9.4 kDa. To be specific, as mentioned above, using a protein marker having a known molecular weight, a calibration curve is prepared while including the range of the protein molecular weight of 2.6 - 9.4 kDa, an elution time corresponding to the range of the protein molecular weight is examined, and the peak area can be measured according to the obtained elution time. Usable protein markers are as mentioned above, and the peak area can be measured according to the instruction manual of the analysis apparatus used. Along with the peak area (A), total peak area (B) obtained by subjecting the peptide mixture of the present invention to gel filtration chromatography is measured, and the proportion of the peak area (peak area rate) relative to the total peak area can be determined using the following formula. The total peak area can also be measured according to the instruction manual of the analysis apparatus used.

$$\text{Peak area rate (\%)} = (A/B) \times 100$$

In the present invention, moreover, a fraction having a range of the protein molecular weight of 2.6 - 9.4 kDa, which is obtained from the peptide mixture of the present invention, can also be utilized. The fraction can be obtained, for example, by a gel filtration method according to a method similar to the method explained for the above-mentioned peptide, and identifying the elution time in the analysis values of a protein marker having a known molecular weight. A fraction eluted from the peptide mixture of the present invention may be directly used or used after applying, to the obtained fraction, a known purification treatment such as concentration under reduced pressure, freeze-drying and the like, utilizing, for example, dialysis membrane, ultrafiltration membrane, microfiltration membrane, reverse osmosis membrane, or ion exchange membrane. These purification treatments can remove unnecessary components, which exert an influence other than the taste enhancing effect, from the obtained fraction. For example, dialysis using purified water can remove phosphoric acid and a salt thereof, as well as other salts from the obtained fraction, whereby the taste enhancing effect can be further enhanced. When dialysis is performed, for example, Tube-O-DIALYZER (manufactured by G-Biosciences), Slide-A-Lyzer (manufactured by Thermo Fisher Scientific Inc.) and the like can be utilized without particular limitation. Ultrafiltration membrane (manufactured by Millipore) and the like for ultrafiltration, MF membrane (manufactured by Millipore) and the like for microfiltration, RO membrane (manufactured by Millipore) and the like for reverse osmosis, and ion exchange membrane (manufactured by SUNACTIS CO., LTD.) and the like for ion exchange can be utilized. Commercially available products can be preferably used as these. The peptide mixture of the present invention may be the fraction per se, a fraction product containing said fraction, or an unfractionated product containing a peptide contained in said fraction.

The peptide mixture of the present invention can enhance the taste of food effectively by addition thereof to the food, and can confer kokumi particularly effectively to the food. Accordingly, the present invention can provide a method of producing a food with an enhanced taste, comprising a step of adding the peptide mixture of the present invention to food, and further, a method for enhancing the taste of food. Furthermore, the present invention can provide a food composition containing the peptide mixture of the present invention, preferably a food composition with an enhanced taste.

In the present specification, "enhance the taste of food" means enhancing one or two or more kinds of the five basic tastes of sweet taste, salty taste, sour taste, bitter taste and *umami* provided by food, and enhancing marginal tastes of the basic tastes such as thickness, growth, mouthfulness, continuity, harmony and the like of the taste. In the present specification, "*kokumi*" is a taste that cannot be expressed by the five basic tastes of sweet taste, salty taste, sour taste, bitter taste and *umami,* and has effects of not only enhancing the basic tastes but also enhancing marginal tastes of the basic tastes such as thickness, growth, continuity, harmony and the like of the taste. In the present specification, an action to enhance the taste of food can also be simply expressed as a "taste enhancing action".

The amount of the peptide mixture of the present invention to be added to food is not particularly limited as long as it can enhance the taste of food, and can be appropriately determined according to the kind of the food to be added to. For example, it can be added in an amount of 0.001 - 99 wt%, preferably 0.01 - 95 wt%, of the food.

The form of the peptide mixture of the present invention to be added to food is not particularly limited, and any form can be taken according to the kind of the food to be added to. For example, it may be a powder, granule, liquid or paste.

The peptide mixture of the present invention can be added to food at any time point such as before production of food (in the starting material), during production of food, after completion of food, immediately before eating food, during eating food and the like.

The peptide mixture itself of the present invention may be added to food as a starting material, or a starting material added with the peptide mixture of the present invention may be added as a starting material of food. For example, the peptide mixture itself of the present invention can be used as a seasoning, or the peptide mixture of the present invention can be added to a seasoning to enhance the taste of the seasoning. Moreover, the peptide mixture of the present invention or a seasoning added with the same can also be used as a starting material of soup and the like.

For example, when a seasoning solution is produced using the peptide mixture of the present invention, the range of an optimal amount of the peptide mixture of the present invention to be used, which varies depending on the seasoning solution to be added with the peptide mixture of the present invention, can be determined by a simple preliminary trial. While the peptide mixture of the present invention can be used directly, typically, a seasoning solution containing 10 wt% or so of the peptide mixture of the present invention is prepared, the seasoning solution is added to food at 0.001 - 5 wt%, mixed and can be used. The concentration of the peptide mixture of the present invention in a seasoning solution can be appropriately determined depending on use, and the concentration of the seasoning solution to be added to food can be controlled according to the concentration.

The mode of utilization of the peptide mixture of the present invention for food is not particularly limited, and may be addition in the form of a seasoning to food and drink, mixing as a starting material of a powder seasoning, a solid seasoning or a liquid seasoning, mixing as a starting material of processed foods and the like. Examples of the food to which the peptide mixture of the present invention is added include, but are not limited to, rice, rice ball, vegetable, pickles, tempura, boiled egg, snack, cereal, fried food, seasonings (seasoning salt, flavor seasoning, *miso,* soy sauce, dipping sauce, sauce, sauce, dressing, mayonnaise etc.), soups (cup soup, soup of instant noodle, etc.), processed foods such as roux and the like, seafood or meat processed products such as *kamaboko, chikuwa, satsuma-age,* ham, sausage and the like, and the like. In the present specification, "food" also includes beverages.

Based on the above-mentioned effects afforded by the peptide mixture of the present invention, the present invention can also provide a taste enhancing agent containing the peptide mixture of the present invention as an active ingredient and foods containing same.

The taste enhancing agent of the present invention characteristically contains the peptide mixture of the present invention, and can enhance the taste of food effectively by addition thereof to the food, and can confer *kokumi* particularly effectively to the food. Therefore, the taste enhancing agent of the present invention can also be utilized as a kokumi-conferring agent. The form of the taste enhancing agent of the present invention is not particularly limited, and may be any such as liquid, paste, powder, granule and the like.

In the present invention, the peptide mixture of the present invention may be directly used as a taste enhancing agent, or constitute a taste enhancing agent in combination with a material other than the peptide mixture of the present invention. When a material other than the peptide mixture of the present invention is used, the concentration of the peptide mixture of the present invention to be contained in the taste enhancing agent is not particularly limited and is, for example, 0.001 - 99 wt%, preferably 0.01 - 95 wt%. Examples of the material other than the peptide mixture of the present invention include additives usable for food such as excipient, disintegrant, moisturizing agent, binding agent, isotonic agent, buffering agent, solubilizing agents, preservative, antioxidant, colorant, corrigent, coagulation agent, pH adjusting agent and the like.

Examples of the excipient include starchy food additive and the like; examples of the disintegrant include cellulose calcium glycolate and the like, examples of the moisturizing agent include calcium stearate and the like, examples of the binding agent include cellulose and the like, examples of the isotonic agent include sorbitol and the like, examples of the buffering agent include sodium acetate and the like, examples of the solubilizing agents include cyclodextrin and the like, examples of the preservative include sodium nitrite and the like, examples of the antioxidant include L-ascorbic acid and the like, examples of the colorant include safflower dye and the like, examples of the corrigent include peppermint oil and the like, examples of the coagulation agent include magnesium chloride and the like, and examples of the pH adjusting agent include sodium lactate and the like. The taste enhancing agent of the present invention can be formulated by adding various additives when needed for preparation making.

The taste enhancing agent of the present invention can be added to various foods, and specific examples thereof are similar to those exemplified above. The amount of the taste enhancing agent of the present invention to be added to food is not particularly limited, and can be appropriately determined according to the kind or dosage form of the food to which the agent is to be added. For example, it can be added such that the content of the peptide mixture of the present invention is 0.001 - 99 wt%, preferably 0.01 - 95 wt%.

The peptide mixture of the present invention can be used as a food taste enhancing agent or a kokumi-conferring agent as mentioned above, as well as a sweet taste enhancing agent, a salty taste enhancing agent, a sour taste enhancing agent, a bitter taste enhancing agent or an umami enhancing agent. All agents can be utilized in a similar mode as the taste enhancing agent of the present invention.

Examples

The present invention is explained in more detail in the following by referring to Examples, which do not limit the technical scope of the present invention. In the Examples, all sensory evaluations were performed by well-trained professional panelists who are engaged in the development work of food.

1. Preparation of hydrolysate of β-conglycinin-containing composition and soybean protein hydrolysate and comparison of taste enhancing effect

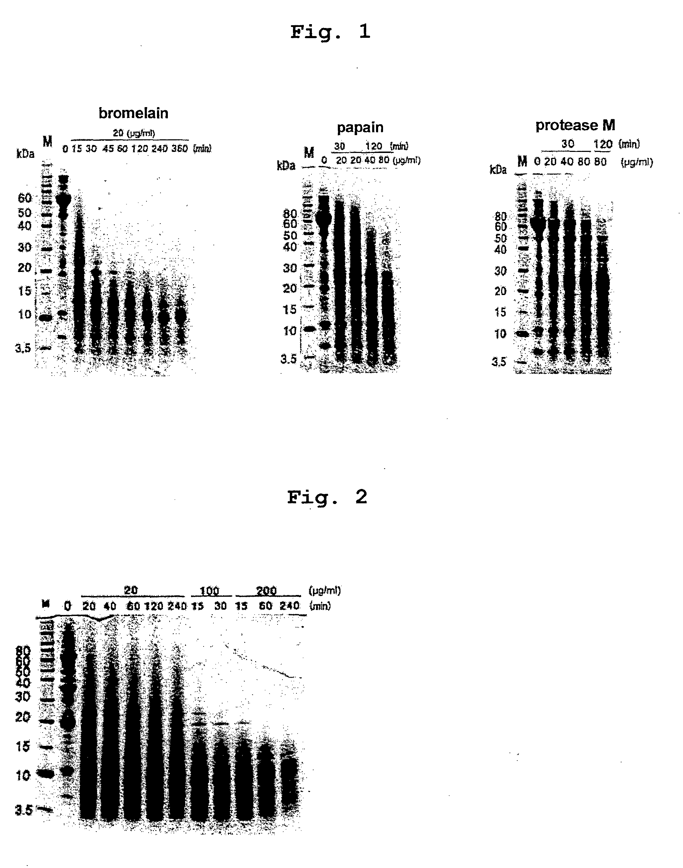

Warm water was added to Lipoff-700 (manufactured by FUJI OIL CO., LTD.) containing not less than 80 wt% of β-conglycinin, to 1.0 wt% thereof, and the mixture was immediately stirred to complete dissolution and cooled to 37°C. Sodium hydroxide solution was added thereto to adjust the pH to 7.0. To 1 L of this solution was added bromelain (manufactured by BIOCON (JAPAN) LTD.), papain (manufactured by BIOCON (JAPAN) LTD.) or protease M (manufactured by Amano Enzyme Inc.) to 20 - 80 μg/mL, and an enzyme reaction was performed for 15 - 360 min. The obtained reaction mixture was boiled to inactivate the enzyme activity of the reaction mixture, after which the reaction mixture was powderized by freeze-drying to give a hydrolysate (hereinafter to be referred to as "β-conglycinin hydrolysate"). Various kinds of β-conglycinin hydrolysates were reduced with an SDS treatment solution containing 2-mercaptoethanol, subjected to SDS electrophoresis using 10% acrylamide gel (40 μg each lane), after which stained with CBB staining solution, and the level of degradation by enzyme was confirmed (Fig. 1).

Using Fujipro-F (manufactured by FUJI OIL CO., LTD.) as soybean protein, warm water was added thereto to 1.0 wt%, and the mixture was immediately stirred, completely dissolved and cooled to 37°C. Sodium hydroxide solution was added thereto, and the mixture was adjusted to pH 7.0. To 1 L of this solution was added bromelain (manufactured by BIOCON (JAPAN) LTD.) to 20 - 200 μg/mL, and an enzyme degradation reaction was performed for 15 - 240 min. The obtained reaction mixture was boiled to inactivate the enzyme activity of the reaction mixture, after which powderized by freeze-drying to give a soybean protein hydrolysate. Various kinds of soybean protein hydrolysates were reduced with an SDS treatment solution containing 2-mercaptoethanol in the same manner as with the β-conglycinin hydrolysate, subjected to SDS electrophoresis using 10% acrylamide gel (40 μg each lane), after which stained with CBB staining solution, and the level of degradation by enzyme was confirmed (Fig. 2).

The level of degradation was confirmed from the SDS electrophoresis pattern, samples for the evaluation of the β-conglycinin hydrolysate and soybean protein hydrolysate were selected as shown in Table 1, and sensory evaluation was performed.

Table 1

β-conglycinin hydrolysate

| enzyme | - | bromelain | | | | | |
|---|---|---|---|---|---|---|---|
| sample | C0 | C1 | C2 | C3 | C4 | C5 | C6 |
| degrading enzyme concentration (μg/ml) | 0 | 20 | 20 | 20 | 20 | 20 | 20 |
| degradation time (min) | 0 | 15 | 30 | 60 | 120 | 240 | 360 |

| enzyme | papain | | | |
|---|---|---|---|---|
| sample | C7 | C8 | C9 | C10 |
| degrading enzyme concentration (μg/ml) | 20 | 20 | 40 | 80 |
| degradation time (min) | 30 | 120 | 120 | 120 |

| enzyme | protease M | | | |
|---|---|---|---|---|
| sample | C11 | C12 | C13 | C14 |
| degrading enzyme concentration (μg/ml) | 20 | 40 | 80 | 80 |

(continued)

| enzyme | protease M | | | |
|---|---|---|---|---|
| sample | C11 | C12 | C13 | C14 |
| degradation time (min) | 30 | 30 | 30 | 120 |

| soybean protein hydrolysate | | | | | | | | | |
|---|---|---|---|---|---|---|---|---|---|
| sample | S0 | S1 | S2 | S3 | S4 | S5 | S6 | S7 | S8 |
| degrading enzyme concentration ($\mu$g/ml) | 0 | 20 | 20 | 20 | 20 | 100 | 100 | 200 | 200 |
| degradation time (min) | 0 | 20 | 40 | 120 | 240 | 15 | 30 | 15 | 60 |

Whole chicken stock (manufactured by Ajinomoto Co., Inc., 20 g) was dissolved in hot water (1 L), and the solution was divided and poured by 100 ml. An evaluation sample (20 mg) was added thereto, and mixed to give chicken soup with a concentration of 200 mg/L for evaluation. Three professional panelists performed a sensory evaluation as to the kokumi strength of the chicken soup according to a rating method. The evaluation by the rating method was performed using the sensory evaluation scores shown in Table 2 as the standard, and the evaluation scores of the three professional panelists were averaged to give evaluation results of various samples. The results of the sensory evaluation are shown in Table 3. As the results of the sensory evaluation, not less than 3 points means "strong taste enhancing effect", not less than 2 points and less than 3 points means "weak taste enhancing effect", and less than 2 points means "no taste enhancing effect".

Table 2
Sensory evaluation score

| | |
|---|---|
| 0.0 | No *kokumi* |
| 1.0 | Slight *kokumi* |
| 2.0 | Weak *kokumi* |
| 3.0 | Strong *kokumi* |
| 4.0 | Very strong *kokumi* |

Table 3

$\beta$-conglycinin hydrolysate

| enzyme | - | bromelain | | | | | |
|---|---|---|---|---|---|---|---|
| sample | C0 | C1 | C2 | C3 | C4 | C5 | C6 |
| sensory evaluation score | 0.0 | 2.0 | 3.2 | 3.0 | 2.4 | 1.0 | 0.8 |

| enzyme | papain | | | |
|---|---|---|---|---|
| sample | C7 | C8 | C9 | C10 |
| sensory evaluation score | 1.7 | 2.0 | 2.0 | 1.8 |

| enzyme | protease M | | | |
|---|---|---|---|---|
| sample | C11 | C12 | C13 | C14 |
| sensory evaluation score | 2.0 | 1.8 | 1.6 | 1.0 |

(continued)

soybean protein hydrolysate

| sample | S0 | S1 | S2 | S3 | S4 | S5 | S6 | S7 | S8 |
|---|---|---|---|---|---|---|---|---|---|
| sensory evaluation score | 0.0 | 1.0 | 1.5 | 0.8 | 0.5 | 0.4 | 0.4 | 0.4 | 0.4 |

From the results shown in Table 3, a strong taste enhancing effect was confirmed in C2 and C3 among the β-conglycinin hydrolysates, and a weak taste enhancing effect was confirmed in C4, C8, C9 and C11. On the other hand, a taste enhancing effect was not confirmed in any sample of the soybean protein hydrolysates.

S2 showing the highest evaluation score among the soybean protein hydrolysates was subjected to the sensory evaluation again according to the above-mentioned method by setting the sample addition concentrations of 400 mg/L, 600 mg/L and 1000 mg/L. However, a taste enhancing effect could not be obtained in any case (Table 4), and when the concentration was not less than 600 mg/L, an unpreferable off-flavor was felt strongly. From the above results, the β-conglycinin hydrolysate was clarified to have a more remarkable taste enhancing effect than the soybean protein hydrolysate.

[Table 4]

| sample | addition concentration (mg/L) | sensory evaluation score |
|---|---|---|
| C2 | 200 | 3.2 |
| S2 | 200 | 1.5 |
| S2 | 400 | 1.9 |
| S2 | 600 | 0.6 |
| S2 | 1000 | 0.4 |

2. Taste enhancing component in β-conglycinin hydrolysate and effect thereof

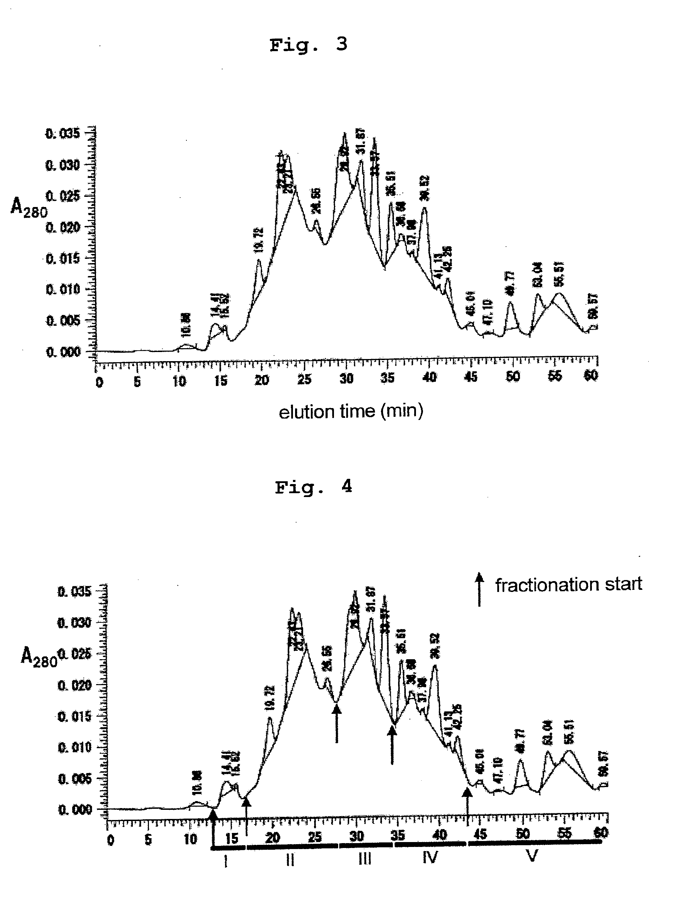

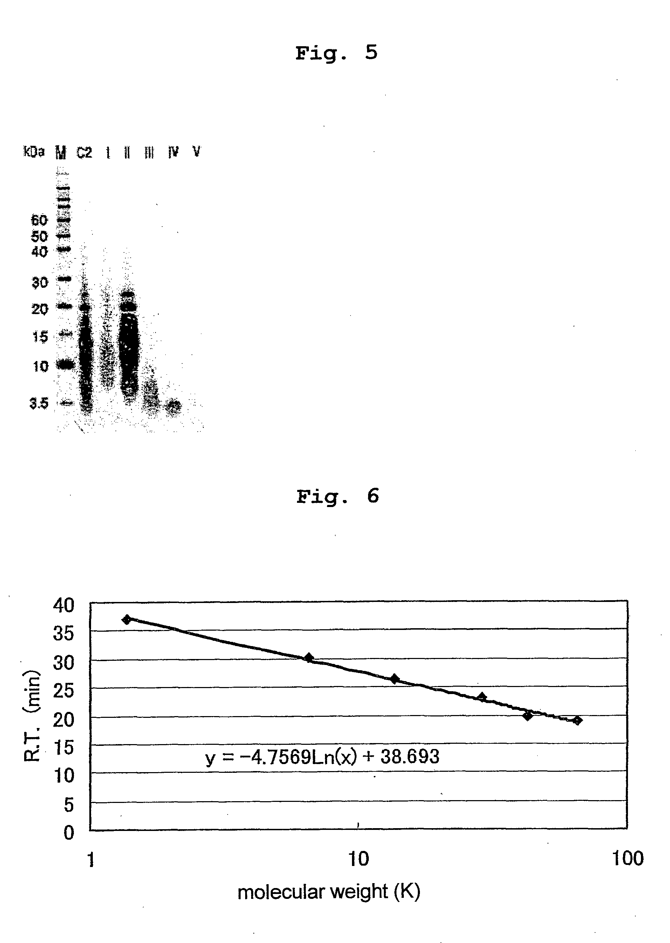

C2 (40 mg) showing the highest evaluation score among the β-conglycinin hydrolysates was subjected to fractionation of protein by gel filtration chromatography using high-performance liquid chromatography system LaChrom (manufactured by Hitachi, Ltd.). The gel filtration chromatography was performed under the following conditions. Column: Superdex 75 100/300 GL (manufactured by GE Healthcare), eluate: 20 mM phosphate buffer (pH 7.0), flow rate: 0.5 mL/min, detection: absorbance at measurement wavelength 280 nm. The results analyzed under these conditions are shown in Fig. 3. Based on the graph shown in Fig. 3, the fractionation start elution time of the fraction was set to 13.0 min, 17.0 min, 28.0 min, 34.0 min or 44.0 min, and fractions I - V were fractionated as shown in Fig. 4. The obtained fractions I - V were reduced with an SDS treatment solution containing 2-mercaptoethanol, subjected to SDS electrophoresis using 10% acrylamide gel, after which stained with CBB staining solution, whereby fractionation was confirmed (Fig. 5). The fractions I - V were dialyzed in purified water using a dialysis membrane Tube-O-DIALYZER (manufactured by G-Biosciences) to remove phosphoric acid and a salt thereof, and powderized by freeze-drying. The weight of the powder of each obtained fraction was as follows (I: 4.3 mg, II: 16.7 mg, III: 6.8 mg, IV: 7.2 mg, V: 9.4 mg).

The powderized fractions I - V were subjected to a sensory evaluation, and which fraction contributes to the taste enhancing effect was examined. Whole chicken stock (manufactured by Ajinomoto Co., Inc., 20 g) was dissolved in 1 L of hot water to prepare chicken soup, and the solution was divided and poured by 200 ml. All of the obtained fractions I - V in powder (I: 4.3 mg, II: 16.7 mg, III: 6.8 mg, IV: 7.2 mg, V: 9.4 mg) were added to the chicken soup and mixed to achieve the same abundance ratio of β-conglycinin hydrolysate before fractionation. Three professional panelists performed a sensory evaluation as to the kokumi strength of the chicken soup according to a rating method. The evaluation by the rating method was performed using the sensory evaluation scores shown in Table 2 as the standard, and the evaluation scores of the three professional panelists were averaged to give evaluation results of fractions I-V. The results of the sensory evaluation are shown in Table 5. As the results of the sensory evaluation, a fraction showing a higher evaluation score was determined to have a stronger taste enhancing effect. In addition, the component contained in a fraction showing the highest evaluation score was determined to contribute most to the taste enhancing effect of β-conglycinin hydrolysate.

[Table 5]

| sample | sensory evaluation score |
|---|---|
| C2 | 3.2 |

(continued)

| sample | sensory evaluation score |
|---|---|
| I | 2.4 |
| II | 2.2 |
| III | 2.8 |
| IV | 2.0 |
| V | 1.5 |

As shown in Table 5, fraction III was found to show the strongest taste enhancing effect, and this fraction among fractions I - V has been determined to contribute most to the taste enhancing effect of β-conglycinin hydrolysate.

3. Range of protein molecular weight of taste enhancing component in β-conglycinin hydrolysate

To determine the range of the protein molecular weight of fraction III showing the highest taste enhancing effect, a relational formula of the elution time and protein molecular weight was calculated by using the same conditions and method as the gel filtration chromatography shown in the above-mentioned 2, and analyzing a protein marker having a known molecular weight. As the protein marker having a known molecular weight, 66 kDa bovine serum albumin (manufactured by Sigma Ltd.), 29 kDa carbonic anhydrase (manufactured by DS Pharma Biomedical Co., Ltd.), 43 kDa ovalbumin (manufactured by Worthington), 13.7 kDa ribonuclease A (manufactured by Nacalai Tesque), 6.5 kDa Aprotinin (manufactured by Nacalai Tesque), and 1.36 kDa cyanocobalamin (manufactured by Wako Pure Chemical Industries, Ltd.) were used. As a result, the relational formula of the protein molecular weight and elution time was calculated as follows (Fig. 6).

$$y = -4.7569 Ln(x) + 38.693$$

x: protein molecular weight (kDa), y: elution time (min)

The range of the protein molecular weight of fraction III (elution time 28.0 - 34.0 min) was determined using the above-mentioned relational formula to find 2.6 - 9.4 kDa. This range was taken as the range of the protein molecular weight of the taste enhancing component.

4. Relationship between the amount of taste enhancing component in β-conglycinin hydrolysate and taste enhancing effect

To examine the relationship between the amount of taste enhancing component in β-conglycinin hydrolysate and the taste enhancing effect, samples C1 - C6 were analyzed using the same conditions and method as the gel filtration chromatography shown in the above-mentioned 2. From the obtained chromatography chart (vertical axis: absorbance, horizontal axis: elution time), the time during which the components in 2.6 - 9.4 kDa, which is the range of the protein molecular weight of the taste enhancing component, are eluted, i.e., peak area (A) eluted during the elution time of 28.0 - 34.0 min, was measured using the analysis software attached to HPLC system manager (manufactured by Hitachi, Ltd.). Also, total peak area (B) was measured from the chart, the peak area rate (%) of the taste enhancing component in samples C1 - C6 was calculated from following formula.

$$peak\ area\ rate\ (\%) = (A/B)\ x\ 100$$

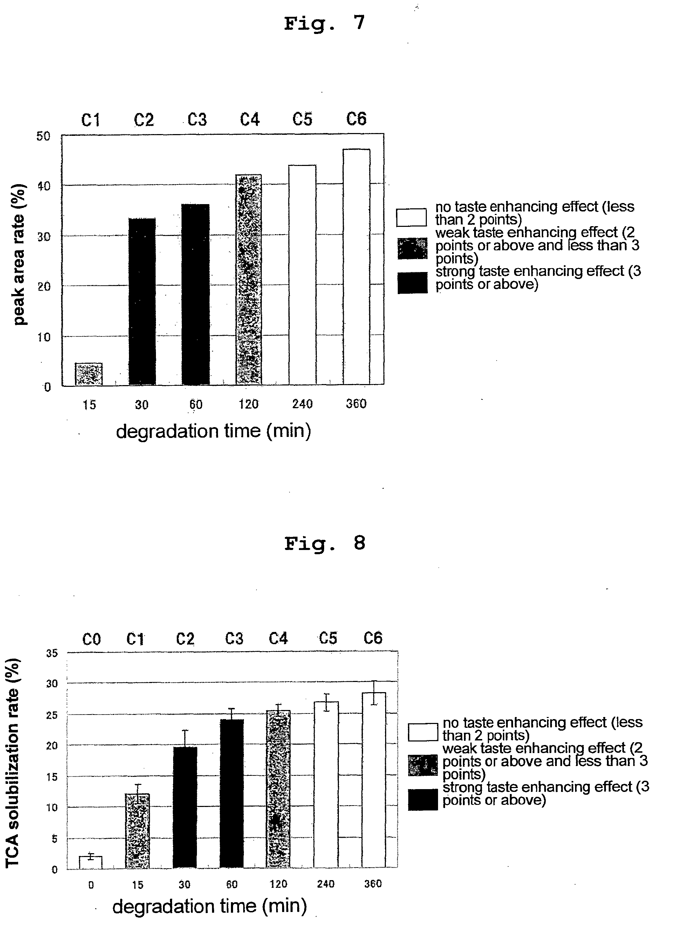

The peak area rates (%) of samples C1 - C6 are shown in Table 6, and the relationship thereof with the taste enhancing effect by sensory evaluation shown in Table 3 is shown in Fig. 7.

[Table 6]

| sample | degradation time (min) | peak area rate (%) |
|---|---|---|
| C1 | 15 | 4.5 |
| C2 | 30 | 33.3 |

(continued)

| sample | degradation time (min) | peak area rate (%) |
|---|---|---|
| C3 | 60 | 36.0 |
| C4 | 120 | 42.0 |
| C5 | 240 | 43.7 |
| C6 | 360 | 46.9 |

As a result, it has been clarified that a taste enhancing effect is confirmed when the peak area rate is 4.5 - 42.0%. On the other hand, when the peak area rate was not less than 43.7%, a taste enhancing effect was not observed.

5. Relationship between taste enhancing effect of β-conglycinin hydrolysate and TCA solubilization rate

To examine the relationship between the taste enhancing effect of β-conglycinin, hydrolysate and the hydrolysis rate, the trichloroacetic acid (TCA) solubilization rate, which is generally used as a hydrolysis rate, was measured for samples C0 - C6. A powder of C0 - C6 was dispersed in water at 1.0 wt%, and sufficiently stirred. To the solution was added an equal amount of 0.44M TCA (manufactured by Nacalai Tesque), and the mixture was sufficiently mixed and incubated at 37°C for 30 min. Thereafter, the precipitate in the solution was removed with filter paper No. 5 (manufactured by ADVANTEC TOYO) to give a filtrate, and the amount of TCA (0.22M) soluble protein was measured by the Lowry method. Also, the total protein amount of each β-conglycinin hydrolysate was measured by the Lowry method. Using the measured TCA soluble protein amount and the total protein amount, the proportion of the amount of the TCA soluble protein to the total protein amount was calculated and the TCA solubilization rate was determined. The TCA solubilization rates of C0 - C6 are shown in Table 7, and the relationship thereof with the taste enhancing effect by sensory evaluation shown in Table 3 is shown in Fig. 8.

[Table 7]

| sample | degradation time (min) | TCA (0.22M) solubilization rate (%) |
|---|---|---|
| C0 | 0 | 2.0 |
| C1 | 15 | 12.1 |
| C2 | 30 | 19.5 |
| C3 | 60 | 24.0 |
| C4 | 120 | 25.4 |
| C5 | 240 | 26.8 |
| C6 | 360 | 28.2 |

As a result, it has been clarified that a taste enhancing effect is confirmed when the TCA solubilization rate is 12.1 - 25.4%. On the other hand, when the TCA solubilization rate was not more than 2.0% or not less than 26.8%, a taste enhancing effect was not observed.

6. Taste enhancing effect of hydrolysate of β-conglycinin containing composition

Warm water was added to Lipoff-700 (manufactured by FUJI OIL CO., LTD.) containing not less than 80 wt% of β-conglycinin to 1.0 wt% thereof, and the mixture was immediately stirred to complete dissolution and cooled to 37°C. Sodium hydroxide solution was added thereto to adjust the pH to 7.0. To 1 L of this solution was added trypsin (manufactured by Novozyme) or aroase (manufactured by YAKULT PHARMACEUTICAL INDUSTRY CO., LTD.), which is a different kind of enzyme from the above, to 20 - 80 μg/mL as shown in Table 8, and an enzyme reaction was performed for 30 - 120 min. The obtained reaction mixture was boiled to inactivate the enzyme activity of the reaction mixture, after which the reaction mixture was powderized by freeze-drying to give a hydrolysate.

[Table 8]

| enzyme | trypsin | | aroase | |
|---|---|---|---|---|
| sample | C15 | C16 | C17 | C18 |
| degrading enzyme concentration (μg/ml) | 20 | 80 | 20 | 80 |
| degradation time (min) | 30 | 120 | 120 | 30 |

Whole chicken stock (manufactured by Ajinomoto Co., Inc., 20 g) was dissolved in hot water (1 L), and the solution was divided and poured by 100 ml. Evaluation samples C15 - C18 (20 mg) shown in Table 8 were added thereto, and mixed to give chicken soup with a concentration of 200 mg/L for evaluation. Three professional panelists performed a sensory evaluation as to the kokumi strength of the chicken soup according to a rating method. The evaluation by the rating method was performed using the sensory evaluation scores shown in Table 2-as the standard. The results are shown in Table 9.

[Table 9].

| sample | C15 | C16 | C17 | C18 |
|---|---|---|---|---|
| sensory evaluation score | 1.4 | 1.8 | 2.0 | 1.8 |

From the results shown in Table 9, a weak taste enhancing effect was confirmed in evaluation samples C15 - C18.

7. Taste enhancing effect of hydrolysate of soybean with high β-conglycinin content

Commercially available soybean "NAYA" (manufactured by Prograin), and "Nanahomare" (Nagano Vegetable and Ornamental Crops Experiment Station, The Hokuriku Crop Science, 2010, vol.45, 61-64) which is a soybean with high β-conglycinin content containing about 2-fold amount of β-conglycinin as compared to general soybeans, were each ground in a powder by a coffee mill (manufactured by OSAKA CHEMICAL Co., Ltd.), and subjected to a deoiling treatment using Soxhlet (manufactured by Chitose Kagaku) and n-hexane (manufactured by JUNSEI CHEMICAL CO., LTD.) as a solvent. After the deoiling treatment, they were dried to give a soybean extract and "Nanahomare" extract (hereinafter to be referred to as "soybean extract with high β-conglycinin content"). Warm water was added to the obtained extract to 1.0 wt%, and the mixture was immediately stirred, suspended and cooled to 37°C. To 1 L of this solution was added bromelain to 20 - 80 μg/mL, and an enzyme reaction was performed for 30 min. The obtained reaction mixture was boiled to inactivate the enzyme activity of the reaction mixture, after which the reaction mixture was powderized by freeze-drying to give a hydrolysate (hereinafter to be referred to as "enzyme degradation product of soybean extract" or "enzyme degradation product of soybean extract with high β-conglycinin content").

A *kokumi*-conferring effect by the addition of each sample to a low-fat food was confirmed according to the method described in WO2008/139945. To be specific, low-fat milk (manufactured by Takanashi milk products Co., Ltd.) was divided and poured by 60 ml, an evaluation sample (12 mg) was added thereto, and mixed to give low-fat milk having a concentration of the evaluation sample of 200 mg/L. Three professional panelists performed a sensory evaluation as to the kokumi strength of the low-fat milk according to a rating method. The evaluation by the rating method was performed using the sensory evaluation scores shown in Table 2 as the standard.

[Table 10]

| evaluation sample | sensory evaluation score |
|---|---|
| No additive | 0.0 |
| soybean extract | 0.9 |
| soybean extract with high β-conglycinin content | 1.1 |
| enzyme degradation product of soybean extract | 1.8 |
| enzyme degradation product of soybean extract with high β-conglycinin content | 2.8 |

From the results shown in Table 10, the strongest taste enhancing effect was confirmed in an enzyme degradation product of soybean extract with high β-conglycinin content, and a soybean extract with high β-conglycinin content before enzymatic degradation did not show a taste enhancing effect.

Industrial Applicability

According to the present invention, it has been clarified that a particular peptide mixture obtained by hydrolyzing a composition containing β-conglycinin effectively enhances the taste of food. The peptide mixture of the present invention is useful in the food field, and use of the peptide mixture of the present invention can effectively enhance the taste of food, and can particularly effectively confer kokumi to food.

This application is based on patent application No. 2011-083155 (filing date: April 4, 2011) filed in Japan, the contents of which are encompassed in full herein.

**Claims**

1. A method of producing a food with an enhanced taste, comprising a step of adding a peptide mixture obtained by hydrolyzing, with an enzyme, a composition comprising β-conglycinin as a main component, which mixture comprises a peptide having a protein molecular weight in a range of 2.6 - 9.4 kDa as measured by a gel filtration method, and shows a trichloroacetic acid (0.22M) solubilization rate of 12.1 - 25.4%.

2. The production method according to claim 1, wherein the peak area of the peptide having a protein molecular weight in the range of 2.6 - 9.4 kDa as measured by a gel filtration method is 4.5 - 42.0% of the total peak area.

3. The production method according to claim 1 or 2, wherein the hydrolysis is performed under the conditions of pH 2 - 11 and/or 4 - 70°C.

4. The production method according to any one of claims 1 - 3, wherein the composition comprises not less than 80 wt% of β-conglycinin.

5. The production method according to any one of claims 1 - 4, wherein the composition is produced from a soybean with a high content of β-conglycinin as a starting material.

6. The production method according to claim 5, wherein the soybean with a high content of β-conglycinin is a soybean species containing 65 mg/g - 400 mg/g of β-conglycinin in a dry weight.

7. A method of producing a food with an enhanced taste, comprising a step of adding a peptide mixture obtained by grinding and deoiling a soybean with a high content of β-conglycinin to give an extract, and hydrolyzing the extract with an enzyme under the conditions of pH 2 - 11 and/or 4 - 70°C.

8. The production method according to any one of claims 1 - 7, wherein the enzyme is one or more kinds selected from the group consisting of bromelain, papain, protease M, trypsin and aroase.

9. The production method according to any one of claims 1 - 8, wherein the peptide mixture is added at 0.001 - 99 wt%.

10. A food composition with an enhanced taste, which is obtained by the production method according to any one of claims 1 - 9.

11. The food composition according to claim 10, which is a seasoning.

12. A method for enhancing a taste of food, comprising a step of adding a peptide mixture obtained by hydrolyzing a composition comprising β-conglycinin as a main component with an enzyme.

13. The method according to claim 12, wherein the composition comprises not less than 80 wt% of β-conglycinin.

14. The method according to claim 12 or 13, wherein the peptide mixture comprises a peptide having a protein molecular weight in a range of 2.6 - 9.4 kDa as measured by a gel filtration method.

15. The method according to any one of claims 12 - 14, wherein the peptide mixture has a trichloroacetic acid (0.22M) solubilization rate of 12.1 - 25.4%.

16. The method according to any one of claims 12 - 15, wherein the enzyme is one or more kinds selected from the group consisting of bromelain, papain, protease M, trypsin and aroase.

17. The method according to any one of claims 12 - 16, wherein the composition is prepared from a soybean with a high content of β-conglycinin as a starting material.

18. The method according to any one of claims 12 - 17, wherein the method for enhancing a taste is a method for conferring *kokumi.*

19. A peptide mixture obtained by grinding and deoiling a soybean with a high content of β-conglycinin to give an extract, and hydrolyzing the extract with an enzyme under the conditions of pH 2 - 11 and/or 4 - 70°C.

20. A taste enhancing agent comprising a peptide mixture obtained by hydrolyzing, with an enzyme, a composition comprising not less than 80 wt% of β-conglycinin, which mixture comprises a peptide having a protein molecular weight in a range of 2.6 - 9.4 kDa as measured by a gel filtration method, and shows a trichloroacetic acid (0.22M) solubilization rate of 12.1 - 25.4%, or the peptide mixture according to claim 19.

21. A food composition comprising a peptide mixture obtained by hydrolyzing, with an enzyme, a composition comprising not less than 80 wt% of β-conglycinin under the conditions of pH 2 - 11 and/or 4 - 70°C, which mixture

    (1) comprises a peptide having a protein molecular weight in a range of 2.6 - 9.4 kDa as measured by a gel filtration method,
    (2) shows a peak area of the peptide, which has a protein molecular weight in the range of 2.6 - 9.4 kDa as measured by a gel filtration method, of 4.5 - 42.0% of the total peak area,
    and
    (3) shows a trichloroacetic acid (0.22M) solubilization rate of 12.1 - 25.4%.

## Fig. 1

## Fig. 2

## Fig. 3

elution time (min)

## Fig. 4

↑ fractionation start

I   II   III   IV   V

Fig. 5

Fig. 6

$$y = -4.7569 \text{Ln}(x) + 38.693$$

molecular weight (K)

## Fig. 7

## Fig. 8

<table>
<tr><td colspan="2" align="center">**INTERNATIONAL SEARCH REPORT**</td><td>International application No.<br>PCT/JP2012/059209</td></tr>
</table>

A.   CLASSIFICATION OF SUBJECT MATTER

*A23L1/227*(2006.01)i, *A23L1/221*(2006.01)i

According to International Patent Classification (IPC) or to both national classification and IPC

B.   FIELDS SEARCHED

Minimum documentation searched (classification system followed by classification symbols)

A23L1/227, A23L1/221

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

JSTPlus/JMEDPlus/JST7580(JDreamII)

C.   DOCUMENTS CONSIDERED TO BE RELEVANT

| Category* | Citation of document, with indication, where appropriate, of the relevant passages | Relevant to claim No. |
|---|---|---|
| X,Y<br>A | WO 2010/078462 A1  (SOLAE, L.L.C./NOVOZYMES<br>A/S),<br>08 July 2010 (08.07.2010),<br>paragraphs [0065], [0090]<br>& JP 2012-513772 A     & EP 2384124 A<br>& CA 2747603 A     & SG 172823 A<br>& MX 2011007107 A | 19,21<br>1-18,20 |
| X,Y<br>A | JP 2008-237127 A  (Fuji Oil Co., Ltd.),<br>09 October 2008 (09.10.2008),<br>abstract; claims; paragraphs [0011], [0017];<br>examples<br>(Family: none) | 19,21<br>1-18,20 |

☒  Further documents are listed in the continuation of Box C.          ☐  See patent family annex.

| | |
|---|---|
| \*    Special categories of cited documents:<br>"A"    document defining the general state of the art which is not considered to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search<br>    02 July, 2012 (02.07.12) | Date of mailing of the international search report<br>    10 July, 2012 (10.07.12) |
|---|---|
| Name and mailing address of the ISA/<br>    Japanese Patent Office | Authorized officer |
| Facsimile No. | Telephone No. |

Form PCT/ISA/210 (second sheet) (July 2009)

| | INTERNATIONAL SEARCH REPORT | International application No. |
|---|---|---|
| | | PCT/JP2012/059209 |

**C (Continuation).   DOCUMENTS CONSIDERED TO BE RELEVANT**

| Category* | Citation of document, with indication, where appropriate, of the relevant passages | Relevant to claim No. |
|---|---|---|
| <u>X,Y</u><br><u>A</u> | JP 2001-069920 A  (Fuji Oil Co., Ltd.),<br>21 March 2001 (21.03.2001),<br>abstract; claims; paragraphs [0016], [0019] to<br>[0020]; examples<br>& JP 2001-69920 A       & US 6303178 B1<br>& EP 976331 A2          & CN 1256088 A | <u>19,21</u><br>1-18,20 |
| <u>X,Y</u><br><u>A</u> | JP 11-221024 A  (Fuji Oil Co., Ltd.),<br>17 August 1999 (17.08.1999),<br>abstract; claims; paragraphs [0007], [0012],<br>[0013], [0020]; examples<br>(Family: none) | <u>19,21</u><br>1-18,20 |
| A | JP 11-089519 A  (Fuji Oil Co., Ltd.),<br>06 April 1999 (06.04.1999),<br>(Family: none) | 1-21 |
| A | JP 9-313111 A  (Fuji Oil Co., Ltd.),<br>09 December 1997 (09.12.1997),<br>& US 6126973 A          & EP 797927 A1<br>& DE 69703311 D         & DE 69703311 T<br>& ES 2152066 T          & CN 1183915 A | 1-21 |
| A | JP 9-313110 A  (Fuji Oil Co., Ltd.),<br>09 December 1997 (09.12.1997),<br>& US 6022702 A          & EP 797928 A1<br>& DE 69700759 D         & DE 69700759 T<br>& ES 2139420 T          & CN 1168770 A | 1-21 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1464928 B
- WO 2004096836 A
- JP 3417350 B
- WO 2006132273 A
- WO 2008139945 A
- JP 2011083155 A

**Non-patent literature cited in the description**

- *Journal of Agriculture and Food Chemistry,* 2007, vol. 55, 6712-6719
- *Journal of Biological Chemistry,* 2010, vol. 285 (2), 1016-1022
- *Journal of Atherosclerosis and Thrombosis,* 2006, vol. 13 (5), 486-490
- *Journal of Agriculture and Food Chemistry,* 1984, vol. 32, 486-490
- *Biochemical and Molecular Action of Nutrients,* 2003, vol. 133, 352-357
- **THANH et al.** *Journal of Agriculture and Food Chemistry,* vol. 24, 1117-1121
- *Journal of Agriculture and Food Chemistry,* 1992, vol. 40, 941-944
- **SAITO et al.** *Bioscience, Biotechnology and Biochemistry,* 2001, vol. 65, 884-887
- Nagano Vegetable and Ornamental Crops Experiment Station. The Hokuriku Crop Science, 2010, vol. 45, 61-64